(19) Europäisches Patentamt
European Patent Office
Office européen des brevets

(11) Veröffentlichungsnummer: **0 276 481**
**A2**

(12) **EUROPÄISCHE PATENTANMELDUNG**

(21) Anmeldenummer: **87119287.8**

(22) Anmeldetag: **29.12.87**

(51) Int. Cl.⁴: **C08G 18/80**, C08G 18/32, A01N 47/38, A01N 47/40, B27K 3/15, B27K 5/00, C07D 235/32, C09D 5/14

(30) Priorität: **12.01.87 AT 41/87**

(43) Veröffentlichungstag der Anmeldung: **03.08.88 Patentblatt 88/31**

(84) Benannte Vertragsstaaten: **BE CH DE ES FR GB IT LI NL SE**

(71) Anmelder: **VIANOVA KUNSTHARZ AKTIENGESELLSCHAFT**
**Postfach 191 Leechgasse 21**
**A-8011 Graz(AT)**

(72) Erfinder: **Awad, Rami-Raimund, Dipl.-Ing.**
**Papiermühlgasse 28**
**A-8020 Graz(AT)**
Erfinder: **Rauch-Puntigam, Harald, Dr.**
**Hochsteingasse 21**
**A-8010 Graz(AT)**
Erfinder: **Morre, Peter**
**Triesterstrasse 125**
**A-8073 Feldkirchen(AT)**
Erfinder: **Tümmler, Peter, Dr.**
**Grillparzerstrasse 6**
**A-8010 Graz(AT)**

(74) Vertreter: **Majcen, Hildegard, Dr.**
**Vianova Kunstharz AG Patentabteilung**
**Leechgasse 21 Postfach 191**
**A-8011 Graz(AT)**

(54) **Verfahren zur Herstellung von kunstharzverträglichen Fungizidpräparaten vom N-(2-Benzimidazol)-Carbamid -säure-Alkylester-Typ.**

(57) Die Erfindung betrifft ein Verfahren zur Herstellung von kunstharzverträglichen N-(2-Benzimidazol)-alkylcarbamate enthaltenden Fungizidpräparaten durch Verknüpfung des Fungizids über eine durch Feuchtigkeit rückspaltbare Carbamoylbrücke mit einem in Holzlasuren oder -imprägnierungen einsetzbaren, in organischen Lösemitteln oder, nach Neutralisation in Wasser löslichen Bindemittel. Das Präparat kann direkt verwendet oder anderen Harzen zugesetzt werden.

EP 0 276 481 A2

Xerox Copy Centre

# Verfahren zur Herstellung von kunstharzverträglichen Fungizidpräparaten vom N-(2-Benzimidazol)-Carbamidsäure-Alkylester-Typ.

Alkylester der N-(2-Benzimidazo)-Carbamidsäure der Formel

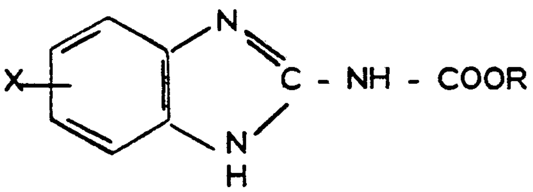

wobei R einen Alkylrest oder einen Alkoxyalkylrest und X ein Wasserstoff-oder ein Halogenatom oder eine Nitro-oder eine Alkylgruppe darstellt, sind aus der Literatur als Fungizide bzw. als Zwischenprodukte für die Herstellung von Fungiziden bekannt. Ihre Herstellung wird beispielsweise in der US-PS 3,637,733 beschrieben.

Ein wesentlicher Nachteil dieser Produkte ist bekannterweise ihre Schwerlöslichkeit in allen üblichen Lösemitteln, wie dies z. B. für das 2-(Methoxycarbonylamino)-benzimidazol (abgekürzt als MBC bezeichnet) in ULLMANN, Band 12, Seite 9/10 angegeben wird, wodurch ihre Verteilung im Anwendungsmedium sehr erschwert wird. Handelsprodukte dieses Fungizidtyps sind daher in der Regel Umsetzungsprodukte des MBC, wobei sich vor allem Reaktionsprodukte mit Mono-oder Diisocyanaten als besonders geeignet herausgestellt haben (siehe z B. die US-PS 3,631,176; die US-PS 3,970,668 oder die DE-OS 26 41 759).

Wie in ULLMANN (s.o.) angegeben wird, zerfällt die Harnstoffverbindung - wahrscheinlich durch die Einwirkung von Feuchtigkeit - unter Rückbildung von MBC.

Für spezielle Anwendungen, wie in Holzlasuren, Holzimprägnierungen u. ä., sind jedoch auch diese Anwendungsformen wenig geeignet, da die Verbindungen nur in speziellen Lösemitteln in ausreichendem Maße löslich und mit den eingesetzten Kunstharzen nicht verträglich sind.

Es bestand daher die Aufgabe, eine für diesen Einsatzzweck brauchbare Form dieser Wirkstoffe zu finden.

Durch die vorliegende Erfindung wird die Aufgabe dadurch gelöst, daß ein N-(2-Benzimidazol)-alkylcarbamat über eine modifizierte Diisocyanatverbindung an das Kunstharz chemisch gebunden wird. Durch diesen Schritt werden die durch die - schlechte Löslichkeit bzw. Unverträglichkeit bedingten Verteilungsschwierigkeiten überwunden.

Die vorliegende Erfindung betrifft demgemäß ein Verfahren zur Herstellung von kunstharzverträglichen Fungizidpräparaten vom N-(2-Benzimidazol)-Carbamidsäure-Alkylestertyp in Form von 1-Carbamoylderivaten, welches dadurch gekennzeichnet ist, daß man ein N-(2-Benzimidazol)-alkylcarbamat mit einem Diisocyanat, dessen NCO-Gruppen unterschiedliche Reaktivität aufweisen, in äquimolaren Mengen umsetzt und die freien NCO-Gruppen, gegebenenfalls über ein Monoalkanolmonoalkylamin und/oder ein Dialkanolamin und/oder ein Hydroxyalkyloxazolidin und/oder eine Hydroxycarbonsäure, mit entsprechenden funktionellen Gruppen eines Polykondensations-oder Polyadditionsharzes zur Reaktion bringt.

Die Erfindung betrifft weiters die Verwendung der in dieser Weise hergestellten Fungizidpräparate als anteiliges oder alleiniges Bindemittel für Anstrichmittel, insbesonders in Holzlasuren und Holzimprägnierungen und ähnlichen Fungizide enthaltenden Schutzanstrichen.

Wie bereits erwähnt, werden als fungizid-wirksame N-(2-Benzimidazol)-Carbamidsäure-Alkylester Verbindungen der allgemeinen Formel

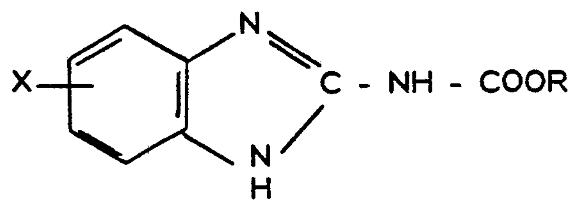

eingesetzt, wobei R einen Alkylrest oder einen Alkoxyalkylrest mit 1 bis 4 C-Atomen und X ein Wasserstoff-oder ein Halogenatom oder eine Nitro-oder eine Alkylgruppe darstellt. Die Reaktion mit der bevorzugt reagierenden NCO-Gruppe des Diisocyanats erfolgt literaturgemäß mit dem Imidazolwasserstoff unter Bildung eines Harnstoffderivates.

Beim erfindungsgemäßen Verfahren wird in erster Stufe ein Diisocyanat, dessen NCO-Gruppen unterschiedliche Reaktivität aufweisen, unter milden Temperaturbedingungen, d. h. bei max, 60 Grad C mit dem N-(2-Benzimidazol)-Carbamidsäure-Alkylester in weitgehend äquimolaren Mengen umgesetzt. In der Praxis hat sich das 2-(Methoxycarbonylamino)-benzimidazol (MBC) als besonders vorteilhaft erwiesen. Gegebenenfalls wird durch einen geringfügigen Überschuß an MBC die teilweise Umsetzung aller Diisocyanat-Moleküle gesichert.

Als Diisocyanate werden die bekannten asymmetrischen Verbindungen, wie das Isophorondiisocyanat, 2,4-Toluylendiisocyanat oder Trimethylhexamethylendiisocyanat eingesetzt.

Das mit der Benzimidazolverbindung halbblockierte Diisocyanat kann nun über seine freie NCO-Gruppe direkt mit den Hydroxylgruppen eines Polykondensations-oder eines Polyadditionsharzes reagiert werden.

Als Polykondensationsharze für diese direkte Umsetzung werden Polyesterharze oder Alkydharze eingesetzt, welche eine entsprechende Anzahl von Hydroxylgruppen aufweisen, die für die Reaktion mit dem Fungizid-Diisocyanat-Reaktionsprodukt zur Verfügung stehen. Bevorzugt werden solche Harze verwendet, die bereits für sich in Holzlasuren oder Imprägnierungen eingesetzt werden. Insbesonders handelt es sich dabei um mittel- bis langölige Alkydharze, wie sie dem Verbraucher in großer Zahl zur Verfügung stehen.

Zur direkten Umsetzung können auch Maleinsäureanhydrid-Adduktverbindungen von längerkettigen, olefinisch ungesättigten Verbindungen, wie von trocknenden Ölen, von diese Öle aufbauenden ungesättigten Fettsäuren oder von niedermolekularen Dienpolymerisaten, wie handelsüblichen Polybutadienölen, herangezogen werden, wenn deren Anhydridgruppen zur Einführung von Hydroxylgruppen partiell mit Diolen aufgeschlossen werden. Vorteilhafterweise wird dabei zuerst ein Teil der Anhydridgruppen mit Wasser oder Monoalkoholen reagiert, um eine doppelseitige Reaktion der Diole möglichst hintanzuhalten. Als Monoalkohole werden dabei vorzugsweise (Poly)-glykolether eingesetzt.

Das mit der Benzimidazolverbindung halbblockierte Diisocyanat kann aber auch nach Reaktion mit einem Alkanolamin, einem Hydroxyalkyloxazolidin oder einer anderen analog reagierenden Verbindung direkt mit den Anhydridgruppen einer der genannten Maleinsäureanhydrid-Additionsverbindungen zur Reaktion gebracht werden.

Als Alkanolamine können dabei sekundäre Monoamine, die entweder eine oder zwei Alkanolgruppen aufweisen, eingesetzt werden. Bei den Hydroxyalkyloxazolidinen erfolgt die NCO-Reaktion mit der Hydroxylgruppe des Hydroxyalkylrestes; die Reaktion mit Anhydridgruppen erfolgt über die durch die Aufspaltung des Oxazolidinringes entstehenden Hydroxyl-bzw. wahrscheinlich auch über die Methylengruppe.

Um die Reaktion mit Oxirangruppen zu ermöglichen, kann als Zwischenglied auch eine Hydroxycarbonsäure eingesetzt werden.

Als Polyadditionsharze können in gleicher Weise auch Copolymere, welche die gewünschten funktionellen Gruppen, wie Hydroxyl-, Carboxyl-, Anhydrid-oder Oxirangruppen aufweisen, eingesetzt werden.

Die Harze können entweder in organischen Lösemitteln löslich sein oder bei entsprechendem Aufbau nach Neutralisation ihrer ionisierbaren Gruppen wasserlöslich sein. Für den Einsatz als Holzschutzanstriche werden in vielen Fällen wasserverdünnbare Produkte bevorzugt.

Der in das Harz eingebaute Fungizidanteil hängt von der notwendigen Wirkstoffkonzentration ab. Es können aber auch Harze mit einem maximal erreichbaren Wirkstoffanteil hergestellt werden, welche als Konzentrat anderen Harzen in entsprechender Menge zugesetzt werden.

Die Formulierung, Herstellung und Anwendung solcher Holzschutzanstriche, wie Lasuren oder Imprägnierungen sind dem Fachmann bekannt bzw. der Fachliteratur zu entnehmen.

Die folgenden Beispiele erläutern die Erfindung, ohne sie in ihrem Umfang zu beschränken. Alle Angaben in Teilen oder Prozenten beziehen sich, soferne nichts anderes angegeben ist, auf Gewichtseinheiten.

(a) <u>Halbblockierung von Isophorondiisocyanat mit 2-(Methoxycarbonylamino)-benzimidazol (MBC)</u>

Zu einer Suspension von 45 g (0,23 Mol) MBC in 130 g Tetrahydrofuran (THF) werden bei 30 bis 35 Grad C 48 g (0,22 Mol) Isophorondiisocyanat innerhalb einer Stunde zugetropft. Nach Ende der Zugabe wird die Temperatur auf 55 bis 60 Grad C erhöht und der Reaktionsablauf mittels Dünnschichtchromatographie verfolgt. Als Laufmittel wird eine Mischung aus gleichen Teilen THF und Toluol (RF/MBC = 0,7 RF/IPDI = 0,9 RF/Produkt = 0,5) verwendet. Nach 2 Stunden ist der NCO-Wert auf 9,8 % gesunken. Der berechnete Wert beträgt 10,2 %. Das Produkt weist einen Festkörpergehalt von 41,7 % auf.

(b) <u>Halbblockierung von 2,4-Toluylendiisocyanat mit MBC</u>

Zu einer Lösung aus 38 g (0,22 Mol) 2,4-Toluylendiisocyanat in 38 g THF wird eine Suspension aus 45 g (0,23 Mol) BMC in 130 g THF bei 30 bis 35 Grad C innerhalb einer Stunde zugetropft. Nach Ende der Zugabe wird die Temperatur auf 50 bis 55 Grad C erhöht und so lange gehalten, bis der NCO Wert auf 11 bis 11, 5 % gesunken ist. Das Produkt weist einen Festkörpergehalt von 33 % auf und wird unmittelbar weiterverarbeitet.

<u>Beispiel 1:</u>

500 g eines Adduktes aus 50 % Leinöl, 40 % dehydratisiertem Rizinusnöl und 10 % Maleinsäureanhydrid mit einer Säurezahl von 114 mg KOH/g werden bei 60 Grad C mit 60 g Diethylenglykolmonobutylether bis zu einer Säurezahl von

etwa 70 mg KOH/g reagiert. Anschließend werden 8 g Monoethylenglykol zugesetzt und die Veresterung bis zu einer Säurezahl von 57 mg KOH/g weitergeführt. Nach Kühlen auf 30 bis 35 Grad C werden 60 g Fungizid-Isocyanatvorprodukt (a) innerhalb 1 Stunde zugetropft und anschließend wird eine Temperatur von 60 bis 65 Grad C bis zu einem NCO-Wert von 0 gehalten. Das Produkt weist einen Festkörpergehalt von 88,7 % und eine Grenzviskositätszahl von 7,6 ml/g ($CHCl_3$/20 Grad C) auf. Das Produkt ist nach Neutralisation von 75 bis 80 % der freien Carboxylgruppen mit Triethylamin oder $NH_3$ mit Wasser verdünnbar. Eine 20%ige Lösung ist klar. Bei Lagerung der verdünnten Lösung entsteht nach ca. 3 Tagen eine Trübung. Das Produkt weist, bezogen auf Festharz, einen MBC-Anteil von 4,9 Gew.-% auf.

Zur Verwendung als Einlaßgrund wird das Harz in üblicher Weist mit einem für Wasserlacke geeigneten Kombinationssikkativ versetzt und mit Triethylamin zu 80 % (bezogen auf die Säurezahl) neutralisiert. Vor der Applikation wird der Lack mit Wasser, gegebenenfalls unter Mitverwendung organischer Hilfslösemittel, auf die gewünschte Viskosität verdünnt.

Beispiel 2:

500 g des in Beispiel 1 eingesetzten Maleinsäure-Öl-Adduktes werden bei 60 Grad C mit 41,3 g Diethylenglykolmonobutylether reagiert bis eine Säurezahl von 53 mg KOH/g erreicht ist. Nach Zusatz von 19,4 g 1,2-Propylenglykol wird die Veresterung bis zu einer Säurezahl von 50 mg KOH/g weitergeführt. Nach Kühlen auf 30 bis 35 Grad C werden 265,5 g des Fungizid-Isocyanat-Vorproduktes (b), 33%ig, innerhalb einer Stunde zugetropft und bei 60 Grad C bis zu einem NCO Wert von 0 reagiert. Das Produkt weist einen Festkörpergehalt von 78,5 % auf.

Das Produkt wird in der gewünschten Menge einem handelsüblichen wasserlöslichen Lackbindemittel, welches nach Herstellerangabe zur Formulierung von Holzlasuren geeignet ist, zugesetzt und gemeinsam mit diesem weiterverarbeitet. Die Verdünnung mit Wasser erfolgt vor der Applikation.

Beispiel 3:

Für eine fungizidhaltige Holzlasur auf Lösungsmittelbasis wird eine Alkydharz in folgender Weise umgesetzt.

1000 g einer 95%igen Lösung in Testbenzin eines Tallölfettsäurealkyds mit einer Öllänge von ca. 78 % (berechnet als Triglycerid), einer Säurezahl von weniger als 10 mg KOH/g und einer Hydroxylzahl von ca. 35 mg KOH./g wird mit 119g der Fungizidkomponente (a) bei 50 bis 60 Grad C umgesetzt, bis der NCO-Wert auf 0 gesunken ist. Es entsteht eine klare Lösung, welche auch beim weiteren Verdünnen mit Testbenzin keine kristallinen Ausscheidungen zeigt. Die Herstellung der Holzlasur erfolgt in üblicher Weise.

Beispiel 4: Verknüpfung über ein Alkanolamin.

Zu 10 g (95 mMol) Diethanolamin und 10 g THF werden 93.6 g (95 mMol) MBC-IPDI-Vorprodukt (a), 41,7%ig, portionsweise bei 30 bis 35 Grad C zugegeben und es wird die Reaktion bis zu einem NCO-Wert von 0 geführt. Der entstandene weiße kristalline Niederschlag wird abfiltriert und zweimal aus Diethylether umkristallisiert. Die angenommene Struktur MBC-IPDI-N-($CH_2$-$CH_2$-OH)$_2$ wurde durch Aufnahme eines IR-Spektrums bestätigt. Die Substanz hat einen Schmelzpunkt von 179 - 185 Grad C.

100 g (0,1 Mol bezogen auf MSA) des im Beispiel 1 eingesetzten Addukts werden mit 5 g (9,6 mMol) des oben hergestellten Vorproduktes bei 80 bis 90 Grad C umgesetzt bis eine Säurezahl von 52 mg KOH/g erreicht ist. Nach Zugabe von 12 g (74 mMol) Diethylenglykolmonobutylether wird bis zum Erreichen der Halbestersäurezahl von 48 mg KOH/g weiterverestert.

Das Produkt weist einen Festkörpergehalt von praktisch 100 % und eine Grenzviskositätszahl von 9,1 ml/g ($CHCl_3$/ 20 Grad C) auf. Nach Neutralisation mit Triethylamin ist das Produkt wasserverdünnbar. Es zeigt in verdünnter Lösung nach 2 Tagen eine leichte Trübung.

Beispiel 5: Verknüpfung über Hydroxyalkyloxazolidin

532 g (ca. 0,5 Mol) des Fungizid-Vorproduktes (a) werden innerhalb eine Stunde gleichmäßig bei 30 bis 40 Grad C mit 60 g (0,5 Mol) eines N-2-Hydroxyethyloxazolidins und 40 g THF versetzt und bis zu einem NCO-Wert von 0 reagiert.

60 g des im Beispiel 1 eingesetzten Addukts werden bei 80 bis 90 Grad C mit 6,7 g (5,7 mMol) der oben hergestellten 45%igen Lösung bis zu einer Säurezahl von 62 mg KOH/g reagiert. Nach Zusatz von 18 g (0,1 Mol) Diethylenglykolmonobutylether wird die Umsetzung bis zu einer Säurezahl von 48 mg KOH/g weitergeführt. Das Produkt weist einen Festkörpergehalt von 95 % und eine Grenzviskositätszahl von 9,7 ml/g ($CHCl_3$/20 Grad C) auf.

Nach Neutralisation mit Triethylamin oder Ammoniaklösung ist das Produkt umbegrenzt in Wasser löslich. Die 20%ige wäßrige Lösung zeigt nach 3 Tagen eine leichte Trübung.

## Ansprüche

1. Verfahren zur Herstellung von kunstharzverträglichen Fungizidpräpareten vom N-(2-Benzimidazol)-Carbamidsäure-Alkylestertyp in Form von 1-Carbamoylderivaten, dadurch gekennzeichnet, daß man eine N-(2-Benzimidazol)-alkylcarbamat mit einem Diisocyanat, dessen NCO-Gruppen unterschiedliche Reaktivität aufweisen, in äquimolaren Mengen umsetz und die freien NCO-Gruppen, gegebenenfalls über ein Monoalkolmonoalkylamin und/oder ein Dialkanolamin und/oder ein Hydroxyalkyloxazolidin und/oder eine Hydroxycarbonsäure, mit entsprechenden funktionellen Gruppen eines Polykondensations-oder Polyadditionsharzes zur Reaktion bringt.

2. Verfahren nach Anspruch 1, dadurch gekennzeichnet, daß man das N-(2-Benzimidazol)-alkylcarbamat-monoisocyanat mit den Hydroxylgruppen eines Alkydharzes oder eines partiell mit Diolen umgesetzten Maleinsäureadduktes an trocknende Öle oder Fettsäuren oder Dienpolymere umsetzt.

3. Verfahren nach Anspruch 1, dadurch gekennzeichnet, daß man die NCO-Gruppe des Monoisocyanats zuerst mit dem Monoalkanolmonoalkylamin und/oder dem Dialkanolamin oder dem Hydroxyalkyloxazolidin oder der Hydroxycarbonsäure umsetzt und dann das Reaktionsprodukt mit den entsprechenden funktionellen Gruppen des Polykondensations-oder Polyadditionsharzes reagiert.

4. Verfahren nach den Ansprüchen 1 bis 3, dadurch gekennzeichnet, daß man als N-(2-Benzimidazol)-alkylcarbamat N-(2-Benzimidazol)-methylcarbamat einsetzt.

5. Verwendung der nach den Ansprüchen 1 bis 4 hergestellten Fungizidpräparate als anteiliges oder alleiniges Bindemittel für Anstrichmittel, insbesonders für Holzlasuren und Holzimprägnierungen.